# EUROPEAN PATENT APPLICATION

(11) **EP 3 703 071 A1**
(43) Date of publication of application: **02.09.2020**
(21) Application number: 19160270.5
(22) Date of filing: 01.03.2019
(51) Int. Cl.: G16H 30/20, G16H 40/20

(54) **COLLECTING IMAGE QUALITY FEEDBACK USING DEDICATED DICOM NODES**

(71) Applicant: Siemens Healthcare GmbH, 91052 Erlangen (DE)
(72) Inventor: Lapp, Robert, 90480 Nürnberg (DE)

(57) **Abstract**

The invention provides a method and a system for assessing an image quality of a medical imaging data file. The method comprises the steps of:
a) receiving (S10) a medical imaging data file;
b) assessing (S20) an image quality of the received medical imaging data file;
c) determining (S30) whether the provided quality evaluation for the received medical imaging data file meets a predefined quality criterion;
d) if it is determined (S30) that the image quality does not meet the predefined quality criterion, transmitting (S40) at least the received medical imaging data file to a remote application entity;
e) analyzing (S60) at least one medical imaging data file (1) transmitted to the remote application entity;
f) generating (S70) a result signal based on the analyzing (S60) in step e) and transmitting the generated result signal to at least one recipient.

## Description

The present invention relates to a method for assessing n image quality of a medical imaging data file, to a system for determining an image quality of a medical imaging data file as well as to related computer program products and computer-readable data storage media.

Medical imaging is an increasingly common and increasingly valuable tool for the diagnosis of a large variety of conditions and diseases. Medical imaging comprises such techniques as computed tomography, magnetic resonance imaging, X-ray imaging, positron emission tomography, ultrasound imaging and the like.

When performing medical imaging, a process often designated as "scanning", a large number of factors influences the image quality of the individual medical imaging data files, that is, individual images or image series. For example, a patient moving during a scan which is performed over an extended period of time may cause blurriness, an insufficient application of contrasting agent may lead to a medical imaging data file with not enough contrast between different organs and so on.

If it is quickly determined that a medical imaging data file (or "image" for short) has insufficient quality, in some instances the scan may be repeated. This usually causes significant additional costs but may yield a medical imaging data file with improved quality. Desirably, if the root cause for the poor quality of the original medical imaging data file is known, measures may be taken to improve the quality of the subsequent scan made to replace it.

However, this approach not only requires a swift and decisive determination of the generated medical imaging data file being of poor quality but also substantial knowledge and understanding of all possible root causes. Due to these very high requirements, this is an approach that is not usually performed as a standard operating procedure.

Image quality assessment in the medical imaging domain, that is assessing the quality of a medical imaging data file, is in most cases subjective and nowadays not automated in any clinical practice.

However, there is a need to understand the root causes for cases of insufficient image quality in order to improve overall diagnostic quality. Such causes could be, for example, wrong scan/reconstruction protocol settings, inappropriate parameter adaptations by the executing technologist (often a technical assistant), technical malfunctioning of the medical imaging device (often designed as a "scanner" or "scanning device"), incorrect patient positioning and/or patient movement during scanning.

In order to try to find common causes, reasons and ideally solutions for insufficient image quality, a large number of medical imaging data files is needed. Currently, it is very difficult to collect a sufficient number of medical imaging data files having poor (or: insufficient) quality in order to be able to perform a meaningful analysis.

Currently, no standardized interfaces or easy-to-use means to report insufficient image quality exist. The issues are worsened by the fact that radiological image reading (i.e. the interpretation of a medical imaging data file by a specialist, in particular a radiologist) is performed commonly on a large number and variety of different reading devices, for example picture archiving and communication systems, PACS, client stations, imaging workstations, web clients and so on. This makes collecting the medical imaging data files in sufficient quality even more challenging.

In particular, a manual collection is nowadays no longer easily done because, for example, the reading of the medical imaging data files (or: images) is often distributed over multiple sites (or: reading stations). In other words, the reading is increasingly often spatially separated from the acquisition of the medical imaging data files at an acquisition station. Acquisition stations may comprise, or consist of, appliances or devices which generate the medical imaging data files in/by examining/scanning a patient (such as medical imaging devices) or appliances or devices which acquire the medical imaging data files in other ways such as receiving them from another image source.

A specialist, in particular radiologist, may be situated at site A and may be sent medical imaging data files from a large number of different acquisition sites where medical imaging scanning devices are situated and which expect to receive, from the specialist, the reading of the transmitted medical imaging data files. In this way, the valuable time of a single radiology specialist can be maximally utilized without the need for each acquisition site to have their own specialist on site.

A frequently used standard for generating, labeling and transmitting medical imaging data files is the DICOM standard, wherein DICOM is a registered trademark and is an acronym for Digital Imaging and Communications in Medicine. Whenever the DICOM standard is referred to herein, it shall be understood that especially the DICOM PS3.1 2019a version of the DICOM standard is meant. However, the concepts and ideas described herein may equally apply to previous and even to subsequent versions of this widely used standard. The DICOM standard is available under http://www.dicomstandard.org and is managed by the Medical Imaging and Technology Alliance, a division of the National Electrical Manufacturers Association.

It is one of the objectives of the present invention to provide a method and a system for assessing an image quality of a medical imaging data file, and in particular optionally to be able to provide guidance or assistance in achieving medical imaging data files of a higher image quality.

The above objective is solved by the subject-matter of the independent claims of the present invention. Additional advantageous options and modifications are expressed by the dependent claims.

According to a first aspect of the invention, a computer-implemented method for assessing a quality of a medical imaging data file is provided, comprising the steps of:
a) receiving a medical imaging data file;
b) assessing an image quality of the received medical imaging data file;
c) determining whether the image quality, according to the assessing in step b), of the received medical imaging data file meets a predefined quality criterion;
d) if it is determined that the image quality does not meet the predefined quality criterion, transmitting at least the received medical imaging data file to a remote application entity;
e) analyzing at least one medical imaging data file transmitted to the remote application entity; and
f) generating a result signal based on the analyzing in step e) and transmitting the generated result signal to at least one recipient.

Medical imaging data may comprise, or consist of, medical images or medical image series (both herein designated as medical imaging data files) produced by any known, or even currently unknown, medical imaging technique (or: modality) such as computed tomography, CT, magnetic resonance imaging, MRI, X-ray imaging, positron emission tomography, PET, ultrasound imaging and/or the like.

Assessing the image quality of the received medical imaging data file may comprise, or consist of, a human user performing an image quality evaluation of the medical imaging data file in question and rating the image quality of the received medical imaging data file along a numerical scale.

In step d), preferably, when it is determined that the medical imaging data file does meet the predefined image quality criterion, said medical imaging data file is then not transmitted to the remote application entity, or, in some embodiments, to another remote application entity such as an entity that is configured to receive or collect medical imaging data files with good (or sufficient) image quality.

The remote application entity is designated as "remote" herein to emphasize that the application entity is different from any acquisition station which is configured and adapted to acquire and/or generate medical imaging data files, that is, for example, medical imaging devices.

In some alternative versions of the invention, the remote application entity may be replaced by an on-site application entity carrying out the same function. For example, such an on-site application entity may be integrated into an acquisition station (e.g. a medical imaging device), into a reading station (e.g. a personal computer, PC, or terminal for assessing the image quality of the medical imaging data files) and/or the like. However, the realization of the invention using a remote application entity is preferred.

Thus, the present invention allows using a remote application entity that may be labeled (e.g. by an application entity title, AET) as a "trash bin" as a digital solution, wherein one advantage also lies in the combination of a dedicated server as the remote application entity with dedicated processing of the medical imaging data files transmitted to the remote application entity for an analysis of medical imaging data files with insufficient image quality.

The embodiments of the first aspect make it possible to combine a rating of insufficient image quality of any medical imaging data file with a statistical analysis of root causes which may be indicated by the result signal. A result signal may also comprise the received medical imaging data file and additional information, for example as image comments and/or annotations, based on the analyzing in step e).

The result signal may, additionally or alternatively, provide an indication linking medical imaging data files with insufficient image quality with corresponding imaging protocols that produce any or most problems regarding image quality. The result signal may, additionally or alternatively, suggest technical means to improve the image quality of a re-take of the received medical imaging data file and/or for producing in the future medical imaging data files with improved image quality.

As a further or alternative example, information about an operator of a medical imaging device producing at least one of the medical imaging data files transmitted to the remote application entity may be reviewed and analyzed in step e), and it may be found out as a result that a specific user of the medical imaging device may be in need of additional training. The result signal may then indicate this user and, optionally, a specific training course intended for said user based on the analysis in step e).

As further examples, the result signal may, additionally or alternatively, indicate a specific type of medical imaging device being prone to produce medical imaging data files with poor image quality, for example medical imaging devices of a particular series of a particular manufacturer, possibly in combination with other parameters such as specific modalities, specific examinations, specific scanning protocols and/or the like.

In some advantageous embodiments, the remote application entity is realized as a dedicated server, in particular a dedicated DICOM node. The dedicated server may be part of an intranet which also comprises one or more acquisition stations (e.g. medical imaging devices, or "scanners") that have produced one or more medical imaging data files. However, the dedicated server may also be a remote server that is connected to one or more acquisition stations only by a wide area network (WAN) such as the Internet. When the dedicated server is part of an intranet, advantageously the medical imaging data files do not necessarily have to be de-identified and/or anonymized as they would have to be for the sake of patient's data protection in case that the dedicated server was a remote server.

In some advantageous embodiments, a plurality of medical imaging data files is collected, for example by the application entity and/or by a remote computing device, and are analyzed in step e) for generating the result signal in step f). In this way, common causes for common deficiencies of the plurality of medical imaging data files can be readily found and optionally also addressed. In particular, gathering the plurality of medical imaging data files may comprise receiving, in several steps a), medical imaging data files, in particular from at least two different acquisition stations which may even be located at different sites.

In particular, when the remote application entity is realized as a dedicated server that is reachable via a wide area network, acquisition stations for generating the medical imaging data file may be provided at a plurality of different locations (for example at different hospital or research institute sites), and all of the medical imaging data files acquired and/or generated by the different acquisition stations may then be transmitted in a respective step d) to one and the same remote application entity, in particular dedicated server, preferably dedicated DICOM node.

In some advantageous embodiments, the medical imaging data files are transmitted by the remote application entity (preferably dedicated server, more preferably dedicated DICOM node) to a cloud computing platform.

In systems based on cloud computing technology, a large number of devices is connected to a cloud computing system via the Internet. The devices may be located in a remote facility connected to the cloud computing system. For example, the devices can comprise, or consist of, equipment, sensors, actuators, robots, and/or machinery in an industrial set-up(s). The devices can be medical devices and equipment in a healthcare unit. The devices can be home appliances or office appliances in a residential/commercial establishment.

The cloud computing system may enable remote configuring, monitoring, controlling, and maintaining connected devices (also commonly known as 'assets'). Also, the cloud computing system may facilitate storing large amounts of data periodically gathered from the devices, analyzing the large amounts of data, and providing insights (e.g., Key Performance Indicators, Outliers) and alerts to operators, field engineers or owners of the devices via a graphical user interface (e.g., of web applications). The insights and alerts may enable controlling and maintaining the devices, leading to efficient and fail-safe operation of the devices. The cloud computing system may also enable modifying parameters associated with the devices and issues control commands via the graphical user interface based on the insights and alerts.

The cloud computing system may comprise a plurality of servers or processors (also known as 'cloud infrastructure'), which are geographical distributed, connected with each other via a network. A dedicated platform (hereinafter referred to as 'cloud computing platform') is installed on the servers/processors for providing above functionality as a service (hereinafter referred to as 'cloud service'). The cloud computing platform may comprise a plurality of software programs executed on one or more servers or processors of the cloud computing system to enable delivery of the requested service to the devices and its users.

One or more application programming interfaces (APIs) are deployed in the cloud computing system to deliver various cloud services to the users.

In some advantageous embodiments, the analyzing in step e) is performed using a trained artificial intelligence entity, in particular a trained artificial neural network. The trained artificial intelligence entity can in particular be implemented by a cloud computing platform to which the plurality of medical imaging data files is transmitted by the remote application entity.

In some advantageous embodiments, the plurality of medical imaging data files is transmitted to and/or from the remote application entity using a standardized transmission protocol, in particular a DICOM transmission protocol. In this way, independent of any particular type of acquisition station, in particular medical imaging device, different medical imaging data files may be transmitted and, optionally and advantageously, jointly analyzed in order to provide an even better basis for determining possible root causes for poor image quality.

In some advantageous embodiments, the result signal indicates a recommendation to a user of a medical imaging device on how to obtain a medical imaging data file with improved quality with respect to the quality of the received medical imaging data file produced or generated from that medical imaging device. For example, the result signal may indicate an improved imaging protocol, a change in device properties, a change in patient positioning, a suggestion to restrict movement of the patient, a suggestion to check certain properties of the patient and/or the medical imaging device and/or the like.

In some advantageous embodiments, the result signal is configured to automatically adjust at least one setting of a medical imaging device (for example, an imaging protocol) in order to obtain a medical imaging data file with improved image quality with respect to the image quality of the received medical imaging data file. If a plurality of medical imaging data files is received, during the analyzing in step e), it may be determined whether a result signal of this sort can be generated (for example, because that specific medical imaging device is capable to receive such a result signal) and to generate such a result signal for automatic adjustment only for those medical imaging devices which are capable to be adjusted in this way.

In some advantageous embodiments, step c) comprises assigning the received medical imaging data file to a predefined data folder. For example, a folder labeled "trash" or "bad image" or the like may be provided, and medical imaging data files put into this specifically labeled data folder may be automatically transmitted to the remote application entity. Other variants for determining the image quality may comprise a user manually setting a value for the image quality after reviewing the medical imaging data file, swiping the medical imaging data file on a touchscreen into a specific direction (i.e. swipe right for good image quality and swipe left for bad image quality), selecting a label out of a plurality of available labels (for example "perfect quality", "good quality", "acceptable quality", "poor quality", "inacceptable quality") and/or the like.

In some advantageous embodiments, step b) and/or step c) is performed manually as described in the foregoing. In this way, the assessment is done making the most of the considerable expertise of highly trained users to assess the image quality.

In some advantageous embodiments, step b) and/or step c) is performed automatically, preferably by a trained artificial intelligence entity, more preferably by a trained artificial neural network. For example, an artificial neural network may be trained with labeled images, the images being labeled with values indicating their image quality and in this way the artificial neural network may be trained e.g. in supervised learning to automatically assess the image quality of even unknown medical imaging data files.

In this sense, the present invention also provides a method for training an artificial intelligence entity, in particular an artificial neural network.

In some advantageous embodiments, the method further comprises a step of de-identifying a medical imaging data file after it is transmitted to a remote application entity, in particular by the remote application entity. This may be done for example by a whitelist approach. For instance, when, as is preferred, the DICOM standard is used for transferring medical imaging data files, the medical imaging data file comprises a DICOM header with information about the patient, the study, and so on. The de-identifying may further comprise, or consist of, removing a portion of the image data files, e.g. specific pixels which might otherwise allow some kind of identification of the image data files.

In the whitelist approach, it may be defined which of these DICOM fields will be transmitted by the remote application entity and, conversely, those not mentioned by the whitelist, will not be transmitted. It is preferred that the remote application entity is located in an intranet with at least one, preferably a plurality, of acquisition stations so that the acquisition stations may, without the need for anonymizing/de-identifying, simply transmit the full medical imaging data files to the remote application entity. The remote application entity may then, if necessary, perform the anonymizing/de-identifying and, for example, transmit the anonymized/de-identified medical imaging data files to a computing device (for example a cloud computing platform) for analysis of large quantities of data.

The invention further provides, according to a second aspect, a system for assessing an image quality of a medical imaging data file, comprising:
at least one quality determination device; and
an application entity, preferably remotely located from the at least one quality determination device;
wherein each quality determination device of the at least one quality determination device is configured to:
   - receive a medical imaging data file;
   - enable an assessment of the image quality of the received medical imaging data file;
   - determine whether the image quality for the received medical imaging data file meets a predefined quality criterion; and
   - transmit the received medical imaging data file, if it is determined that the image quality does not meet the predefined quality criterion, to the application entity (and preferably do not transmit the received medical imaging data file to the application entity if it is determine that the image quality does meet the predefined quality criterion);
wherein the application entity has an input interface for receiving the medical imaging data file from each quality determination device of the at least one quality determination device; and
a computing device configured to:
   - analyze at least one medical imaging data file transmitted to the application entity;
   - generate a result signal based on the analyzing (i.e. based on a result of the analysis) and transmit the generated result signal to at least one recipient.

The computing device may be realised as any device, or any means, for computing, in particular for executing a software, an app, or an algorithm. For example, the computing device may comprise a central processing unit (CPU) and a memory operatively connected to the CPU. The computing device may also comprise an array of CPUs, an array of graphical processing units (GPUs), at least one application-specific integrated circuit (ASIC), at least one field-programmable gate array, or any combination of the foregoing.

In some advantageous embodiments, the application entity comprises an anonymizing module configured to anonymize and/or de-identify medical imaging data files received by the remote application entity, in particular by use of a whitelist, with the advantages as described in the foregoing.

According to a third aspect of the present invention, a computer program product is provided which comprises executable program code configured to, and executed by a computing device, perform the method according to an embodiment of the first aspect of the invention.

According to a fourth aspect of the present invention, a non-transitory computer-readable data storage medium is provided which comprises executable program code configured to, and executed by a computing device, perform the method according to an embodiment of the first aspect of the invention.

It shall be understood that any variants, options and modifications described for the method according to the first aspect of the present invention may be equally applied to embodiments of the second, third or fourth aspect of the invention and vice versa.

Further advantageous embodiments will be apparent from the dependent claims as well as from the following specification in combination with the figures.

Further advantageous variants and embodiments are described and comprised in the dependent claims as well as in the following description in combination with the drawings.

### Brief description of the drawings

The invention will be explained in greater detail with reference to exemplary embodiments depicted in the drawings is appended.

The accompanying drawings are included to provide a further understanding of the present invention and are incorporated in and constitute a part of this specification. The drawings illustrate the embodiments of the present invention and together with the description serve to explain the principles of the invention.

Other embodiments of the present invention and many of the intended advantages of the present invention will be readily appreciated as they become better understood by reference to the following detailed description. Like reference numerals designate corresponding similar parts. It shall be understood that method steps are numbered for easier reference but that said numbering does not necessarily imply steps being performed in that order unless explicitly or implicitly described otherwise. In particular, steps may also be performed in a different order than indicated by their numbering. Some steps may be performed simultaneously or in an overlapping manner.
- Fig. 1: shows a schematic flow diagram illustrating a method according to an embodiment of the first aspect of the present invention;
- Fig. 2: shows a schematic block diagram illustrating a system according to an embodiment of the second aspect of the present invention;
- Fig. 3: schematically illustrates a computer program product according to an embodiment of the third aspect of the present invention; and
- Fig. 4: schematically illustrates a data storage medium according to an embodiment of the fourth aspect of the present invention.

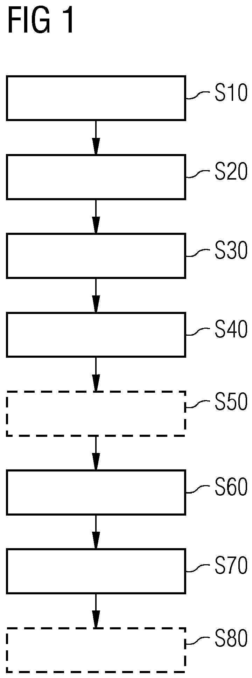

Fig. 1 shows a schematic flow diagram illustrating an embodiment of the method according to the first aspect of the present invention. Accordingly, Fig. 1 illustrates a computer-implemented method for assessing an image quality of a medical imaging data file.

In a step S10, a medical imaging data file (or: medical image) is received, for example from an acquisition station such as a medical imaging device (or: scanner), from a picture archiving and communication system, PACS, and/or the like. It shall be understood and apparent from the foregoing and the following description that step a) may be performed or repeated for a plurality of times for a plurality of medical imaging data files.

In a step S20, the image quality for the received medical imaging data file is assessed. The assessment may be performed manually, that is, by a human user (or: operator), or automatically, for example by an algorithm, preferably by a trained artificial intelligence entity, more preferably by a trained artificial neural network. The medical imaging data file may be received in step S10 by a personal computer, PC or a terminal within an intranet of a hospital or a research institution, over an intranet connection and/or over a secure Internet connection or the like.

The user may then review on a screen of the terminal or PC the received medical imaging data file and rate it, for example by assigning one of a plurality of predefined labels to it, by selecting a numerical image quality value and/or the like for it. The assessing of the image quality may even be performed at the acquisition station itself. For example, the medical imaging data file may be received by a screen or monitor of the medical imaging device itself after the medical imaging data file has been acquired (preferably immediately) so that an operator of the medical imaging device can decide whether the medical imaging has been successful or whether it needs to be repeated straightaway or in the future.

In a step S30, it is determined whether the image quality according to the assessment in step S20 for the received medical imaging data file meets a predefined quality criterion. For example, when the medical imaging data files are to be rated by a numerical scale, a threshold value may be defined which separates numerical values which are deemed acceptable from numerical values for the image quality which are deemed poor or unacceptable, the latter being values that do not meet the predefined quality criterion in this example.

It should be understood that steps S20 and S30 may be closely connected, overlapping or even one and the same. For example, when an operator of a medical imaging device is viewing a medical imaging data file (in particular an image or image series) recently produced by the medical imaging device, the operator may determine that the image quality is insufficient and may proceed to delete said medical imaging data file, that is, to mark said medical imaging data file as "to be deleted", to select it and press a "delete" button, to mark it as "unacceptable" and/or the like. In this way, assessing the image quality may be coincidental with determining that the image quality according to the assessment does not meet the predefined quality criterion.

In a step S40, if it is determined in step S30 that the image quality does not meet the predefined quality criterion, at least the received medical imaging data file is transmitted to a remote application entity. This may be done after a prompt to a user or operator who has performed the determining in step S30 (for example: "Send deleted image for analysis?") or may be performed automatically by any of the actions associated with deleting the medical imaging data file as described above or as known in the prior art.

In other words, a "trash bin" of a client, PC or a terminal for receiving medical imaging data files and assessing their image quality may be linked to the remote application entity. Preferably, the remote application entity is a dedicated server, in particular a dedicated DICOM node. DICOM nodes have the additional advantage that they can process the widely DICOM protocol especially for file transmission and so are able to receive a large variety of medical imaging data files from a large variety of different medical imaging devices produced by different manufacturers, used by different tenants in different jurisdictions and so on.

In an optional step S50, the received medical imaging data file is anonymized and/or de-identified, preferably by the remote application entity itself. As has been discussed in the foregoing, it is especially advantageous if the remote application entity is in the same intranet or otherwise secure internal network as the entity from which the medical imaging data file is received in step S10, in particular as an acquisition station that has generated the medical imaging data file such as a medical imaging device. In that case, it is sufficient if the anonymizing/de-identifying is only performed by the remote application entity and does not have to be performed earlier in the progress of this method.

For example, a hospital (as an example of one type of tenant, or site) may set up a single remote application entity, in particular a dedicated DICOM node, to which all medical imaging data files which do not satisfy (or meet) the predefined quality criterion that have been produced by medical imaging devices of this hospital (or other tenant) can be sent, where they may then be anonymized/de-identified.

In a step S60, at least one medical imaging data file transmitted to the remote application entity is analyzed. The analyzing S60 may be performed by the remote application entity itself. However, preferably, the analyzing S60 is performed by a computing device to which the remote application entity transmits the at least one medical imaging data file transmitted to the remote application entity. As has been discussed before, the computing device may be present in the same intranet as the remote application entity and/or at least one acquisition station, for example medical imaging device for generating the medical imaging data files. In this case, step S50 of anonymizing/de-identifying may be omitted as no patient data are leaving the secure intranet environment.

However, for many applications, it is preferred that there is a database with as many medical imaging data files as possible in order to provide meaningful and statistically relevant analysis. For this reason, it is advantageous if the analyzing S60 is performed by a remote computing device that is external to an intranet comprising the remote application entity.

In order to provide the computing power and data storage capability necessary in cases of large numbers of medical imaging data files, the analyzing S60 may be advantageously performed by a cloud computing platform. In that case, performing step S50 of anonymizing/de-identifying the medical imaging data files is preferred. One and the same computing device which may be, for example, set up and maintained by a digital health service provider, can be configured to receive medical imaging data files from a plurality of remote application entities.

For example, each tenant (for example hospital, research institution, private medical practice and so on) that participates in the method may have set up their own remote application entity, for example in their own intranet, so that their own remote application entity may receive medical imaging data files from all of the acquisition stations and/or medical imaging data sources present at the site of the tenant. Then, each of the remote application entities may transmit, either in bunches or individually, the received medical imaging data files to the computing device, in particular cloud computing platform, for the analyzing S60.

In this way, the database that is used for analyzing the medical imaging data files with poor image quality is richly diverse, including preferably data from multiple tenants, multiple imaging devices, multiple patients and so on in order to be able to ideally find all possible correlations and root causes for the poor image quality.

In a step S70, a result signal based on the analyzing in step S60 is generated and transmitted to at least one recipient.

It will be understood that the at least one recipient may be a single recipient, for example a user, acquisition station or other type of medical imaging data source, which has transmitted a medical imaging data file to the corresponding remote application entity.

For example, the user could be an operator of a medical imaging device and perform an exam to create a medical imaging data file. This medical imaging data file could then be received in step S10 by a PC or terminal (either integrated into the medical imaging device or connected to it by e.g. an intranet connection), where the same user may assess S20 the quality of the medical imaging data file.

Upon determining S30 that the quality is insufficient, the user could then, for example by deleting the file, cause that the file is automatically transmitted S40 to the remote application entity, for example a remote application entity of the hospital site at which the user is present. After the optional anonymization/de-identifying in step S50, the (anonymized) medical imaging data file may then be analyzed S60, for example by a computing device (e.g. a cloud computing platform).

This analyzing may S60, in some advantageous embodiments, comprise determining a likely root cause, or a list of root causes, for poor image quality, each with a corresponding likelihood. In the step S70, a result signal may be generated that comprises or indicates this determined root cause or a list of root causes, and which is then transmitted back to the original user, for example via the remote application entity or over another channel.

Since data about the user of the medical imaging device is not patient-specific, it may be left unaffected by the anonymizing/de-identifying such that enough information about the user, their tenant, their site, their remote application entity and/or the like is remaining within the anonymized medical imaging data file so as to determine where the generated result signal is to be transmitted. The user may then review the data indicated by the result signal and may adjust the medical imaging device accordingly.

As has been discussed before, the generated result signal may be also configured such that it can be transmitted S70 directly to a medical imaging device so as to adjust at least one setting of the medical imaging device automatically in order to improve the image quality, in particular for a medical imaging data file for the same patient and the same exam as the one that produced the poor image quality.

For example, in one very convenient embodiment, the user may acquire the medical imaging data file by performing the medical imaging exam, and steps S10 to S70 may then be performed automatically, optionally followed by an additional step S80 of adjusting, using the generated result signal, the medical imaging device. The adjusting S80 of the medical imaging device may comprise controlling the medical imaging device to output a visual and/or acoustic information or warning signal, controlling the medical imaging device such as to make an adjustment to at least one parameter, in particular an imaging protocol, controlling the medical imaging device to suggest a change in at least one parameter, in particular imaging protocol, to a user, and/or the like.

Due to the large computing power available nowadays, in particular with respect to cloud computing platforms, all of these steps may be performed in very little time so that, to the user, very shortly after having finished performing the medical imaging exam, a signal is sent (for example as part of the generated result signal) which informs the user that now the performed medical imaging exam is to be repeated.

The signal may inform the user that an enclosed list of parameter adjustments is suggested to be made before the new medical imaging exam is performed, or, in the event that the result comprises automatic adjustments, inform the user that an enclosed list of parameter adjustments have been made automatically. In the latter case, the user may be prompted to confirm or deny the automatically made adjustments or changes to the settings or parameters of the medical imaging device.

The medical imaging data files transmitted from the at least one remote application entity to the computing device may also be stored (preferably in their anonymized/de-identified versions) in the computing device, preferably cloud computing platform. Using a client software, for example a web interface, a user (not necessarily a user which has participated in or performed the medical imaging exam) may have a large variety of options on how to analyze and/or manipulate the medical imaging data files collected at the computing device. For example, the user may review images, link them to corresponding protocols, further classify and/or analyze the medical imaging data files stored at the computing device. The computing device (e.g. a cloud computing platform) may advantageously be configured to allow and facilitate any or all of the above functions to users.

The analyzing in step S60 may comprise combining ratings of insufficient quality of the medical imaging data files with statistical analyses of root causes. Automatically by the computing device, for example by a trained artificial intelligence entity, and/or by a user, additional information may be added to the medical imaging data files, for example image comments and/or annotations.

The analyzing S60 of the medical imaging data files may comprise analyzing them with regard to image noise, motion artifacts and/or contrast content. As an example, missing contrast content in a medical imaging data file may be correlated to a corresponding contrast injection protocol.

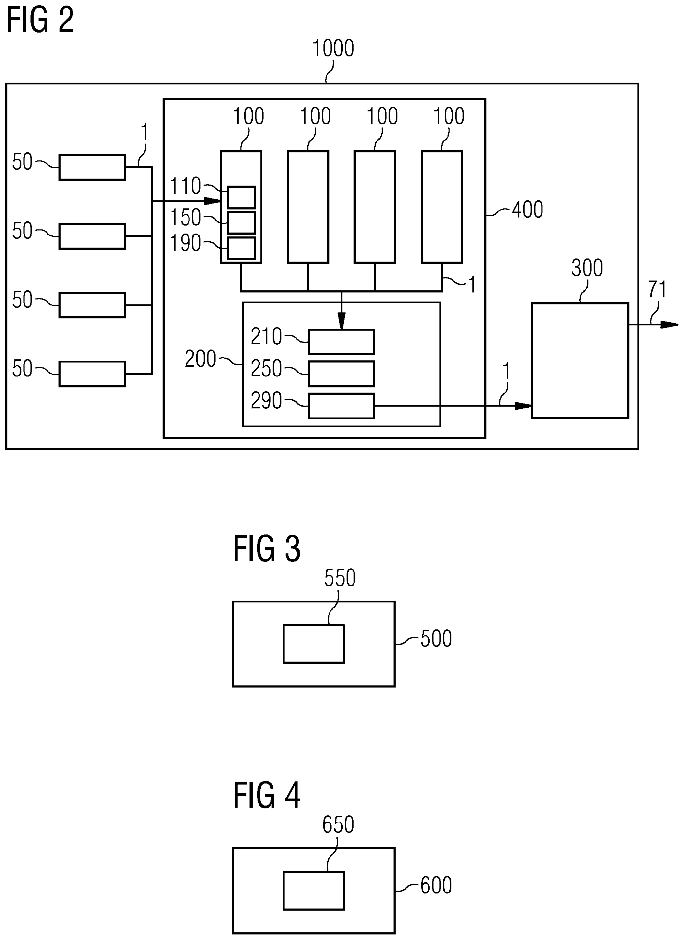

Fig. 2 shows a schematic block diagram illustrating a system 1000 for assessing an image quality of a medical imaging data file 1 according to an embodiment of the second aspect of the present invention. As has been mentioned before, the system 1000 of Fig. 2 is especially suited to perform the method according to embodiment of the first aspect of the present invention, in particular according to the method of Fig. 1. The system 1000 can be modified or improved according to any of the advantageous options, variations and modifications as described with respect to the method according to the first aspect, in particular with respect to the method of Fig. 1 and vice versa.

The system 1000 comprises at least one quality determination device 100 and at least one application entity 200. Each quality determination device 100 of the at least one quality determination device 100 is configured to receive S10 a medical imaging data file 1;
- enable a quality assessment (or: an assessing S20 of image quality) of the received medical imaging data file 1;
- determine S30 whether the image quality for the received medical imaging data file 1 meets a predefined quality criterion; and
- transmit S40, if it is determined that the image quality does not meet the predefined quality criterion, the received medical imaging data file 1 to the application entity 200.

For these tasks, the quality determination device 100 may comprise a corresponding quality assessment module, determination module and transmission module (or: output interface).

The modules may be realized in hardware, such as a circuit or a printed circuit board and/or comprising transistors, logic gates and other circuitry. Additionally, the modules may be at least partially realized in terms of software. Accordingly, the modules may comprise, or be operatively coupled to, a processor and a memory storing a software or a firmware that is executed by the processor to perform the functions of the modules. Signals may be received by an input interface of the modules and signals that the processor of the modules creates may be outputted by an output interface of the modules. The modules may be implemented, at least partially, as a microcontroller, an ASIC, an FPGA and so on.

The quality determination device 100 may be realized as a personal computer PC, a terminal, a mobile device and/or the like. The quality determination device 100 can be configured such that the quality assessment and/or the determining S40 is performed manually by a user, such that it is performed automatically or such that it can be chosen whether it shall be performed automatically or manually. The quality determination device 100 may run a web interface connecting to a server of a digital services provider. In some advantageous embodiments, the quality determination device 100 may be integrated into a medical imaging device.

The quality determination device 100 may be realised as any device, or any means, for computing, in particular for executing a software, an app, or an algorithm. For example, the quality determination device 100 may comprise a central processing unit (CPU) 150 for executing the software, app, or algorithm, and a memory operatively connected to the CPU, an input interface 110 and an output interface 190 (shown in Fig. 2 only for one of the quality determination devices 100).

As schematically illustrated in Fig. 2, a plurality of quality determination devices 100 may be present, in particular on-site of a tenant, for example individual workstations of several radiologists working at the same tenant site. The receiving S10 of the medical imaging data file 1, the determining S30 and/or the transmitting S40 may in particular be performed as has been described with respect to any embodiment of the first aspect of the invention, in particular as has been described in detail with respect to the method of Fig. 1.

The application entity 200 comprises an input interface 210 for receiving the medical imaging data file 1 from each quality determination device 100 of the at least one quality determination device 100. Advantageously, the application entity 200 also has an output interface 290 for outputting any, preferably each, of the medical imaging data files 1 received by the input interface 210, to a computing device 300 of the system 1000. As has been described in the foregoing, the computing device 300 may in particular be a cloud computing platform.

The input interface 210 and/or the output interface 290 may be realized in hardware, such as a circuit or a printed circuit board and/or comprising transistors, logic gates and other circuitry. Additionally, the input interface 210 and/or the output interface 290 may be at least partially realized in terms of software. Accordingly, the input interface 210 and/or the output interface 290 may comprise, or be operatively coupled to, a processor and a memory storing a software or a firmware that is executed by the processor to perform the functions of the input interface 210 and/or the output interface 290.

The application entity 200 may in particular be realized as a dedicated server (comprising e.g. a central computing unit CPU), more preferably as a DICOM node as has been described in more detail in the foregoing with respect to the method according to the first aspect, in particular with respect to the method of Fig. 1.

Thus, preferably, the at least one quality determination device 100 and the remote application entity 200 are located on-site of a tenant and are preferably interconnected via a secure local connection such as an intranet 400. By contrast, the computing device 300, if realized as a cloud computing platform, is located at a remote site and/or in a distributed way over one or more servers.

Especially when the computing device 300 is realized as a cloud computing platform or as another type of remote device (for example, a single server located at a remote site of a services provider), it is advantageous when the application entity 200 comprises a anonymizer/de-identifier module 250 which is configured to anonymize and/or de-identify the medical imaging data files 1 received by the application entity 200 via its input interface 210. The anonymizer/de-identifier module 250 may be configured to perform the anonymizing/de-identifying step S50 as has been described in detail with respect to the method of Fig. 1.

In particular, the anonymizer/de-identifier module 250 may apply a whitelist to each medical imaging data file 1 which specifies which fields of the medical imaging data file 1, for example which fields of a DICOM header, are to be transmitted by the output interface 290 of the application entity 200. The de-identifying may further comprise, or consist of, removing a portion of the image data files, e.g. specific pixels which might otherwise allow some kind of identification of the image data files.

The computing device 300 is configured to:
- analyze S60 at least one medical imaging data file 1 transmitted to the application entity 200;
- generate a result signal 71 based on the analyzing by the computing device 300 and to transmit the generated result signal to at least one recipient. In particular, the computing device 300 may be configured to perform the analyzing S60 and the generating S70 and transmitting of the result signal 71 as has been described with respect to the method according to any embodiments of the first aspect of the invention, in particular with respect to the method of Fig. 1.

The result signal 71 may comprise any or all of the pieces of information that have been described in the foregoing, for example indications of possible means to improve image quality, suggestions to adjust or change settings or protocols of a medical imaging device, control signals performing automatic adjustments of parameters or settings of medical imaging devices and/or the like.

The system 1000 may comprise at least one medical imaging device 50, wherein at least one of the at least one quality determination device 100 is configured to receive medical imaging data files 1 from one, from any combination, or from all of the medical imaging devices 50 of the system 1000. In particular, the medical imaging devices 50 may also be arranged within the intranet 400 such that the security of the personal health information of the patients is guaranteed. If the result signal 71 is configured such as to comprise at least one control signal for automatic adjustment of parameters or settings of a medical imaging device 50, the system 1000 may be set up such that such a result signal 71 is automatically transmitted to any or all of the medical imaging devices 50 of the system 1000.

Fig. 3 schematically illustrates a computer program product 500 comprising executable program code 550. The executable program code 350 may be configured to perform, when executed (e.g. by a computing device), the method according to the first aspect.

Fig. 4 schematically illustrates a non-transitory computer-readable data storage medium 600 comprising executable program code 650 configured to, when executed (e.g. by a computing device), perform the method according to the first aspect.

In the foregoing detailed description, various features are grouped together in one or more examples or examples with the purpose of streamlining the disclosure. It is to be understood that the above description is intended to be illustrative, and not restrictive. It is intended to cover all alternatives, modifications and equivalents. Many other examples will be apparent to one skilled in the art upon reviewing the above specification.

The embodiments were chosen and described in order to best explain the principles of the invention and its practical applications, to thereby enable others skilled in the art to best utilize the invention and various embodiments with various modifications as are suited to the particular use contemplated. In the appended claims and throughout the specification, the terms "including" and "in which" are used as the plain-English equivalents of the respective terms "comprising" and "wherein," respectively. Furthermore, "a" or "one" does not exclude a plurality in the present case.

## Claims

1. A computer-implemented method for assessing an image quality of a medical imaging data file (1), comprising the steps of:
a) receiving (S10) a medical imaging data file (1);
b) assessing (S20) an image quality of the received medical imaging data file (1);
c) determining (S30) whether the image quality for the received medical imaging data file (1) meets a predefined quality criterion;
d) if it is determined (S30) that the image quality does not meet the predefined quality criterion, transmitting (S40) at least the received medical imaging data file (1) to a remote application entity (200);
e) analyzing (S60) at least one medical imaging data file (1) transmitted to the remote application entity (200);
f) generating (S70) a result signal (71) based on the analyzing (S60) in step e) and transmitting the generated result signal (71) to at least one recipient.

2. The method of claim 1,
wherein the remote application entity(200) is realized as a dedicated server.

3. The method of claim 1 or claim 2,
wherein the result signal (71) indicates a recommendation to a user of a medical imaging device (50) on how to obtain a medical imaging data file (1) with improved image quality with respect to the image quality of the received medical imaging data file (1).

4. The method of any of claims 1 to 3,
wherein the result signal (71) is configured to automatically adjust at least one setting of a medical imaging device (50) in order to obtain a medical imaging data file (1) with improved image quality with respect to the image quality of the received medical imaging data file (1).

5. The method of any of claims 1 to 4,
wherein step c) comprises assigning the received medical imaging data file (1) to a predefined data folder.

6. The method of any of claims 1 to 5,
wherein step b) and/or step c) is performed automatically.

7. The method of any of claims 1 to 6,
further comprising de-identifying and/or anonymizing (S50) the medical imaging data file (1) after it is transmitted to the remote application entity (200).

8. The method of any of claims 1 to 7,
wherein a plurality of medical imaging data files (1) is gathered and analyzed (S60) in step e) for generating (S70) the result signal (71) in step f).

9. The method of claim 8,
wherein the plurality of medical imaging data files (1) is transmitted by the remote application entity (200) to a cloud computing platform (300).

10. The method of any of claims 1 to 9,
wherein the analyzing (S60) in step e) is performed using a trained artificial intelligence entity.

11. The method of any of claims 1 to 10,
wherein the medical imaging data file (1) is transmitted to and/or from the application entity (200) using a standardized transmission protocol.

12. A system (1000) for assessing an image quality of a medical imaging data file (1), comprising:
at least one quality determination device (100);
an application entity (200);
wherein each quality determination device (100) of the at least one quality determination device (100) is configured to:
- receive (S10) a medical imaging data file (1);
- enable an assessment (S20) of the image quality of the received medical imaging data file (1);
- determine (S30) whether the image quality for the received medical imaging data file (1) meets a predefined quality criterion; and
- transmit (S40) the received medical imaging data file (1), if it is determined that the image quality does not meet the predefined quality criterion, to the application entity (200);
wherein the application entity (200) has an input interface for receiving the medical imaging data file (1) from each quality determination device (100) of the at least one quality determination device (100); and
a computing device (300) configured to:
- analyze(S60) at least one medical imaging data file (1) transmitted to the application entity (200);
- generate (S70) a result signal (71) based on the analyzing (S60) and transmit the generated result signal (71) to at least one recipient.

13. The system (1000) of claim 12, wherein the application entity (200) further comprises an anonymizer/de-identifier module (250) configured to anonymize and/or de-identify at least one medical imaging data file (1) received by the application entity (200).

14. A computer program product (500) comprising executable program code (550) configured to, when executed, perform the method according to any of claims 1 to 11.

15. A non-transitory computer-readable data storage medium (600) comprising executable program code (650) configured to, when executed, perform the method according to any of claims 1 to 11.
